# EUROPEAN PATENT APPLICATION

(11) **EP 4 603 597 A1**
(43) Date of publication of application: **20.08.2025**
(21) Application number: 24158283.2
(22) Date of filing: 19.02.2024
(51) Int. Cl.: C12Q 1/6876

(54) **AGE DETERMINATION BY DETERMINING DNA METHYLATION LEVELS OF SELECTED CPG SITES**

(71) Applicant: Siemens Healthcare Diagnostics Products GmbH, 35041 Marburg (DE)
(72) Inventor: Fellendorf, Sandra, 50827 Köln (DE); Klatt, Torbjörn, 50374 Erftstadt (DE); Kläver, Ruth, 40721 Hilden (DE); Leenders, Frauke, 42699 Solingen (DE)
(74) Representative: Siemens Healthineers Patent Attorneys

(57) **Abstract**

The present invention relates to a method for determining the age group of a subject, comprising determining the methylation level of a group of methylation sites in a genomic DNA derived from the subject's sample, wherein said group of methylation sites comprises differentially methylated positions (DMPs) corresponding to Illumina probe IDs cg05736768 (SEQ ID NO: 1), cg10741153 (SEQ ID NO: 2) and cg14002960 (SEQ ID NO: 3). Also envisaged is a corresponding kit and a method of forensic DNA analysis of samples derived from male subjects.

## Description

### TECHNICAL FIELD

The present invention relates to a method for determining the age group of a subject, comprising determining the methylation level of a group of methylation sites in a genomic DNA derived from a sample of the subject, wherein said group of methylation sites comprises differentially methylated positions (DMPs) corresponding to Illumina probe IDs cg05736768 (SEQ ID NO: 1), cg10741153 (SEQ ID NO: 2) and cg14002960 (SEQ ID NO: 3). Also envisaged is a corresponding kit, comprising probes for detecting the DMPs corresponding to Illumina probe IDs cg05736768 (SEQ ID NO: 1), cg10741153 (SEQ ID NO: 2) and cg14002960 (SEQ ID NO: 3), a method of forensic DNA analysis of samples derived from male subjects, and the use of DMPs for determining the age group of a subject.

### BACKGROUND

In many application fields, an exact age determination of the individual would have great benefit. However, this is not easy to obtain if only biological samples of this person are available. One exemplary area of application is forensics since often only minimal traces of a biological sample (e.g., blood or saliva) should provide clues to the unknown suspect or missing victim. Here, it would be extremely helpful for the identification and search of the suspect or victim to be able to determine the age range of the person on the basis of a found blood sample, in order to narrow down the possible group of persons as far as possible (Maulani & Auerkari, 2020, Egyptian Journal of Forensic Sciences, 10, 38).

To estimate the age of the producer of a biological sample, different molecular-based methods have been developed, such as telomere repeat length that decreases with increasing age (Blasco, 2005, Nature Review Genetics, 6, 611-622), mRNA mutations that accumulate with increasing age (Weidner et al., 2014, Genome Biology, 15; Zubakov et al., 2016, Genetics, 24, 33-43), T-cell DNA rearrangements (sjTREC) (Zubakov, et al., 2010, Current Biology, 22, PR970-971; Zubakov, et al., 2016) and/or age-dependent deletions of mitochondrial DNA (Cortopassi et al., 1992, PNAS, 89, 7370-7374).

In the last decade, an approach using DNA methylation of selected CpG sites for age estimation has been described based on the finding that DNA methylation patterns change with increasing age and contribute to age related disease (Bocklandt et al., 2011, PLOS, 6, e14821). In particular, it could be shown that the age of individuals can be predicted from DNA methylation analyses in saliva - with an average deviation range of 5.2 years. In subsequent years further studies were published including more than 70 markers in blood (Hannum, et al., 2013, Cell 49(2), 359-367) and 353 CpG sites in multiple tissues (Horvath, S., 2013, Genome Biology, 14:R115, 1-19).

However, many of these approaches display high margin errors, low precision and practical limitations and lack an acceptable accuracy that is useful for the different application fields (Maulani & Auerkari, 2020; Weidner, et al., 2014). Moreover, additional markers could improve predictability in age determination, in particular in specific fields such as forensics.

In view of the above, there is a clear need for further age determination markers and corresponding methods, in particular in the field of forensics and in similar disciplines.

### SUMMARY

The present invention addresses these needs and provides in a first aspect a method for determining the age group of a subject, comprising determining the methylation level of a group of methylation sites in a genomic DNA derived from a sample of the subject, wherein said group of methylation sites comprises differentially methylated positions (DMPs) corresponding to Illumina probe IDs cg05736768 (SEQ ID NO: 1), cg10741153 (SEQ ID NO: 2) and cg14002960 (SEQ ID NO: 3).

The currently claimed methods and uses provide several advantages in comparison to screening approaches of the prior art. To start with, the herein described set of CpG sites is capable of discriminating between a young and an old cohort. A further advantage of the present invention is that current age models can be improved by the proposed set of CpG sites. In addition, a new model for age discrimination can be established using the herein described CpG sites. Moreover, since the herein identified CpG sites allow age discrimination, time- and cost-efficient assays (e.g., PCR-based) can be established to narrow down a larger group of individuals, e.g., for the use in forensics or to supplement Y-chromosomal short tandem repeat (Y-STR) analysis. This clearly overcomes the shortcoming of currently available assays such as Novos age (Novos Labs) and myDNAge (Epimorphy) which require the analysis of several hundreds or thousands of CpG sites, thus being rather unsuitable for forensic testings.

The Y-STR test is a widely used test in forensics and allows distinguishing male from female trace material. Within the Y-STR test, a certain number of variable numbers of short tandem repeats (STR), which are short repeating gene segments, are analyzed on the Y-chromosome. The strictly paternal inheritance of the Y-chromosome allows the assignment to a specific family but fails to specify an individual of the family. The herein described CpG sites can be used in forensics as an extension of the Y-STR and allow further narrowing of a suspect group to a younger (e.g., sons) or older (e.g., father, grandfather) cohort of a family thus facilitating law enforcement accordingly. A further advantage is a significant cost reduction due to the strongly reduced number of DMPs to be analyzed (e.g. 3 CpG sites) in comparison to prior art panels comprising more than 300 methylation sites.

In a preferred embodiment, said determination of the age group of a subject comprises performing a statistical prediction. Said statistical prediction preferably utilizes a machine learning approach such as logistic regression or random forest classification trained on an appropriately representative training cohort.

In a further preferred embodiment, the determination of the age group of a subject comprises a distinction between a younger person, preferably of < 40 years and an older person, preferably of > 60 years, more preferably of > 63 years.

It is further preferred that said sample is a blood, urine, feces, saliva, or liquid biopsy sample, preferably a blood plasma sample.

In further embodiments the group of methylation sites additionally comprises one or more DMPs of Table 2. It is particularly preferred that the group of methylation sites additionally comprises 1, 2, 3, 4, 5, 6, 7, 8, 9, 10, 11, 12, 13, 14, 15, 16, 17, 18 or 19 DMPs of Table 2.

In yet another embodiment, the determination of the methylation level of the methylation sites comprises modifying the genomic DNA molecules to allow for determination of the methylation status of methylation sites, preferably by bisulfite-conversion or enzymatic conversion, and determining the sequence of the molecules, thereby also determining the methylation status of the molecules.

In yet another embodiment, the method comprises a step of reporting the age group of the subject, preferably in an electronic form.

In a further aspect, the present invention relates to a kit comprising probes for detecting the DMPs corresponding to Illumina probe IDs cg05736768 (SEQ ID NO: 1), cg10741153 (SEQ ID NO: 2) and cg14002960 (SEQ ID NO: 3).

In a preferred embodiment the kit according to the invention additionally comprises one or more probes for detecting one or more DMPs of Table 2, preferably 1, 2, 3, 4, 5, 6, 7, 8, 9, 10, 11, 12, 13, 14, 15, 16, 17, 18 or 19 probes binding to cognate DMPs of Table 2, more preferably one or more probes for detecting the DMPs corresponding to Illumina probe IDs cg23479922 (SEQ ID NO: 4), cg03738025 (SEQ ID NO: 5), cg26422458 (SEQ ID NO: 6), cg23746497 (SEQ ID NO: 7), cg03450948 (SEQ ID NO: 8), cg06782035 (SEQ ID NO: 9), cg10997627 (SEQ ID NO: 10), cg18961681 (SEQ ID NO: 11), cg15466862 (SEQ ID NO: 12), cg03323696 (SEQ ID NO: 13), cg25078444 (SEQ ID NO: 14), cg13611347 (SEQ ID NO: 15), cg12071888 (SEQ ID NO: 16), cg15084543 (SEQ ID NO: 17), cg27252696 (SEQ ID NO: 18), cg16065021 (SEQ ID NO: 19), cg26890189 (SEQ ID NO: 20), cg06335867 (SEQ ID NO: 21), or cg04360793 (SEQ ID NO: 22).

In a further aspect, the present invention relates to a method of forensic DNA analysis of samples derived from male subjects, comprising the performance of Y-chromosome short tandem repeat polymorphism (Y-STR) analysis of a subject's sample and determining the methylation level of a group of methylation sites in a genomic DNA derived from a sample of the subject, wherein said group of methylation sites comprises differentially methylated positions (DMPs) corresponding to Illumina probe IDs cg05736768 (SEQ ID NO: 1), cg10741153 (SEQ ID NO: 2) and cg14002960 (SEQ ID NO: 3), wherein the determination of the methylation level allows for the determination of the age group of said subject.

In a preferred embodiment, the group of methylation sites additionally comprises one or more DMPs of Table 2.

In a further aspect, the present invention relates to the use of differentially methylated positions (DMPs) corresponding to Illumina probe IDs cg05736768 (SEQ ID NO: 1), cg10741153 (SEQ ID NO: 2) and cg14002960 (SEQ ID NO: 3), optionally further comprising one or more of DMPs corresponding to Illumina probe IDs cg23479922 (SEQ ID NO: 4), cg03738025 (SEQ ID NO: 5), cg26422458 (SEQ ID NO: 6), cg23746497 (SEQ ID NO: 7), cg03450948 (SEQ ID NO: 8), cg06782035 (SEQ ID NO: 9), cg10997627 (SEQ ID NO: 10), cg18961681 (SEQ ID NO: 11), cg15466862 (SEQ ID NO: 12), cg03323696 (SEQ ID NO: 13), cg25078444 (SEQ ID NO: 14), cg13611347 (SEQ ID NO: 15), cg12071888 (SEQ ID NO: 16), cg15084543 (SEQ ID NO: 17), cg27252696 (SEQ ID NO: 18), cg16065021 (SEQ ID NO: 19), cg26890189 (SEQ ID NO: 20), cg06335867 (SEQ ID NO: 21), or cg04360793 (SEQ ID NO: 22) for the determining the age group of a subject.

In a preferred embodiment, the determination of the age group of a subject is performed within the process of forensic DNA analysis comprising the performance of Y-chromosome short tandem repeat polymorphism (Y-STR) analysis.

### BRIEF DESCRIPTION OF THE DRAWINGS

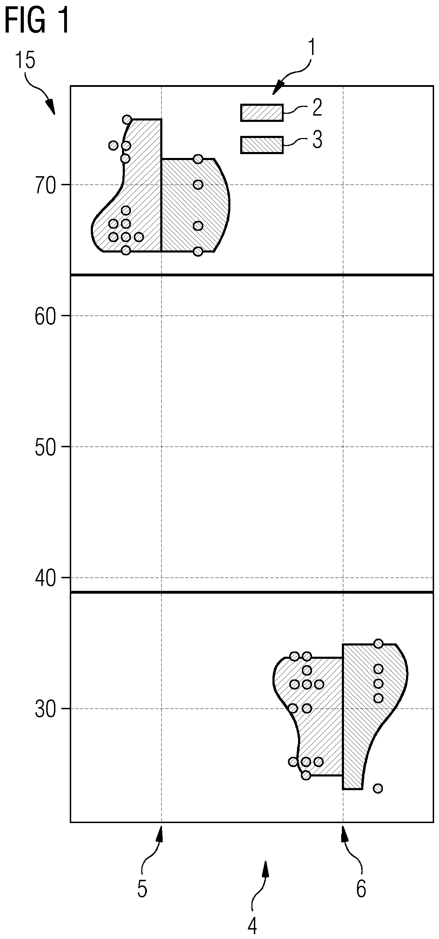

FIG. 1 shows the age distribution of samples used for a bioinformatic assessment according to an embodiment of the invention. Indicated is the gender **(1),** being either male **(2)** or female **(3).** Further indicated is the age group **(4),** being either old **(5)** or young **(6).** On the left hand side the age **(15)** is shown.

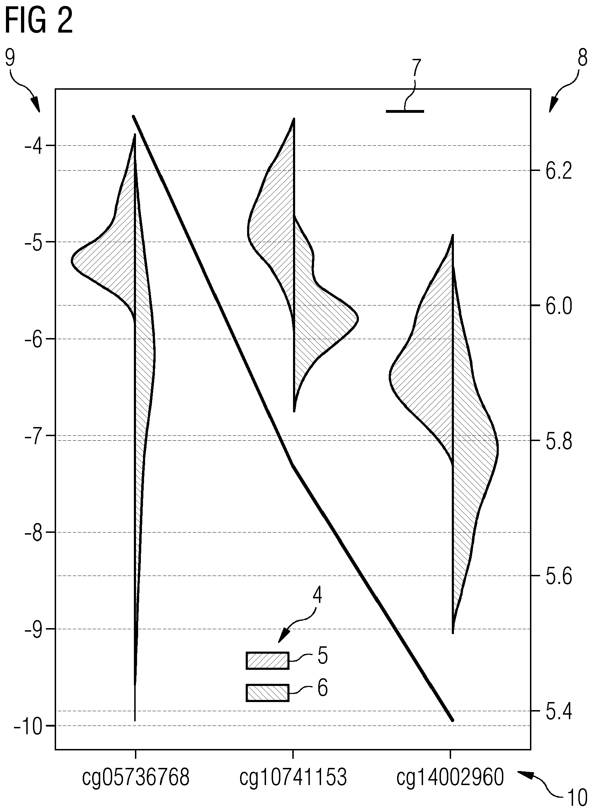

FIG. 2 depicts a distribution of m values, as a metric to measure methylation levels, of Kolmogorov Smirnov (KS)-significant CpG sites. Positive m-values indicate that more molecules are methylated than unmethylated and negative m-values indicate that more molecules are unmethylated than methylated. Indicated is age group **(4),** being either old **(5)** or young **(6).** Further, the determined p-value (depicted as black line with legend **(7)).** The specific CpG sites **(10)** are provided as Illumina ID numbers. On the left hand side the m-value **(9)** is shown, on the right hand side the log10 (pks) of the KS tests **(8)** is depicted.

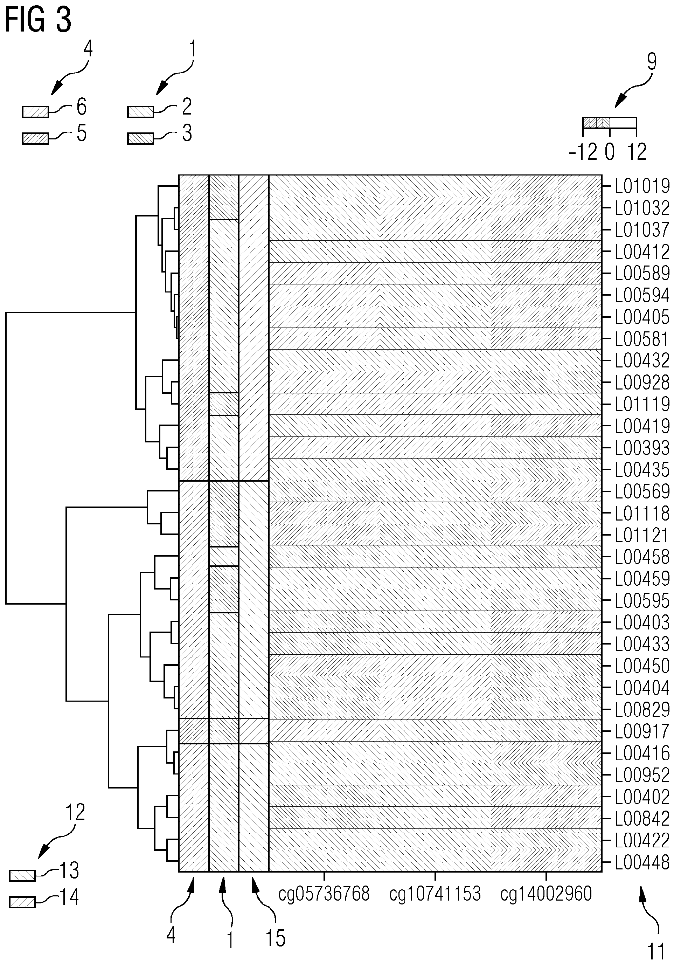

FIG. 3 shows a Ward clustering on CpG sites with Kolmogorov-Smirnov test between old and young samples. Indicated is the gender **(1),** being either male **(2)** or female **(3).** Further indicated is the age group **(4),** being either old **(5)** or young **(6).** On the left hand side the age **(15)** is shown. Also depicted is the m-value **(9),** as well as an age (gradient) **(12)** being either youngest (24 observations) **(13)** or oldest (75 observations) **(14).** On the right hand side the library-ID is depicted **(11).**

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms " (i) ", "(ii)", "(iii)" or " (a) ", "(b)", "(c)", "(d)", or "first", "second", "third" etc. and the like in the description or in the claims, are used for distinguishing between similar or structural elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e., the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a method for determining the age group of a subject, comprising determining the methylation level of a group of methylation sites in a genomic DNA derived from a sample of the subject, wherein said group of methylation sites comprises differentially methylated positions (DMPs) corresponding to Illumina probe IDs cg05736768 (SEQ ID NO: 1), cg10741153 (SEQ ID NO: 2) and cg14002960 (SEQ ID NO: 3). The term "methylation site" as used herein relates to the specific sites in a genomic DNA sequence which are modified by the addition of a methyl group to a cytosine which is converted to a 5-methylcytosine, or, in certain situations, the removal of a methyl group from the 5-methylcytosine. The methylation of DNA is a form of epigenetic modification which has the capability of altering the expression of genes and other elements and may accordingly have an effect on, e.g., silencing of tumor suppressor genes and/or increasing the expression of oncogenes, etc. Some of these epigenetic modifications have been shown to be correlated with age.

Typically, the methylation site is a 5'-cytosine-phosphate-guanine-3' (CpG) dinucleotide site. The methylation sites are typically distributed throughout the genome and not confined to expressed genes. In many cases, methylation sites are located in promoter or regulatory regions. The analysis of the methylation status at certain methylation sites as defined herein is based on specific techniques for the detection of methyl groups. Typically, DNA molecules to be analyzed for the presence of their methylation status are specifically processed to preserve the methylation status information. Such processing may be based on techniques such as bisulfite conversion or enzymatic conversion. The "bisulfite conversion" comprises the treatment of DNA molecules with bisulfite which converts cytosine to uracil but does not convert 5-methylcytosines. Subsequent amplification, which turns uracil to thymine, is usually carried out using PCR or methylation-specific PCR (MSP). In PCR amplification uracil is recognized as thymine and the opposite strand is filled in with adenine due to complementary base pairing, while cytosines are paired with guanines in bisulfite-untreated stretches. This results in two DNA sequences (converted Cs for unmethylated and unconverted Cs for methylated), of which the converted one has C->U transitions at the unmethylated sites and is then paired with A instead of G in DNA sequencing. In the alternative method of "enzymatic conversion" instead of a chemical conversion of unmethylated cytosines an enzymatic conversion is performed. Typically, a set of two enzymes is used. For example, the enzymes TET2 and T4-BGT are used to convert 5-methylcytosines into products that cannot be deaminated by the enzyme APOBEC. In a second reaction, the enzyme APOBEC deaminates unmodified cytosines by converting them to uracils. Further analysis steps correspond to the bisulfite approach described above.

The term "age group" as mentioned herein means a group of subjects whose age can be determined up to a certain level of accuracy, preferably into "young" and "old". In preferred embodiments, the age group "young" is a group of subjects having a chronological age of up to about 40 years. In further preferred embodiments the age group "old" is a group of subjects having a chronological age of above about 60 years, more preferably of above about 63 years. The method according to the present invention, based on the determination of the methylation level of the group of methylation sites comprising the DMPs cg05736768 (SEQ ID NO: 1), cg10741153 (SEQ ID NO: 2) and cg14002960 (SEQ ID NO: 3), thus allows to determine whether a subject belongs to the age group young, i.e. is 40 years old or younger, or belongs to the age group old, i.e. is 60 years, preferably 63 years old, or older. Subjects having an age of about 40 to about 60 years cannot be allocated to one of the age groups according to the present invention.

A correlation of the above mentioned methylation sites with age groups within the context of the present invention may thus be based on the presence of a differentially methylated position (DMP), which shows a methylation status variation in a genomic DNA derived from a subject's sample if said subject belongs to a "young" age group as defined herein above in comparison to a subject belonging to an "old" age group as defined herein above. The differential methylation pattern underlying the present invention and enshrined in the DMPs of Table 1 is not restricted to a simple pattern of non-methylated in the one age group and methylated in the other age group, or vice versa, but reflects combinatorial methylation situations (methylated or unmethylated) involving all methylation sites depicted in Table 1, or in Table 1 and 2.

The group of core methylation sites to be analyzed in order to implement the present method as mentioned above can be derived from the following Table:

**Table 1**

| **No.** | **CpG Site ID (DMP)** | **Chromosome** | **Start position** | **End position** | **Gene** | **SEQ ID NO:** |
|---|---|---|---|---|---|---|
| 1 | cg05736768 | 8 | 104467020 | 104467021 | DPYS | 1 |
| 2 | cg10741153 | 19 | 28793446 | 28793447 | MAN1A2P1 | 2 |
| 3 | cg14002960 | 18 | 34159459 | 34159460 | NOL4 | 3 |

In the following Table 1a the genomic sequence surrounding the CpG sites of the DMPs of Table 1 is provided in detail (with the CpG site being marked):

**Table 1a**

| **No.** | **CpG Site ID (DMP)** | **SEQ ID NO:** | **Sequence with marked CpG Site (underlined)** |
|---|---|---|---|
| 1 | cg05736768 | 1 | Ggccgggcgggcttggggtgccctcctgcaaggtccc caccgacagcccccgagctctgcctcaggctgcaaat ccggagcc |
| 2 | cg10741153 | 2 | Ccgcccgcgcttcccgacgctgccaggcaggctcccg ggacgggcagagctccgaggctgcctccgggcagtca cgtccaac |
| 3 | cg14002960 | 3 | Tgggtctctcggaggagcaggagacgcgggctcgaag ccgcgggcctgaccctgatccgtggatgccgccgccg ccttctcc |

In certain embodiments of the method of the present invention the group of methylation sites additionally comprises one or more DMPs of Table 2:

**Table 2**

| **No.** | **CpG Site ID (DMP)** | **Chromosome** | **Start position** | **End position** | **Gene** | **SEQ ID NO:** |
|---|---|---|---|---|---|---|
| 4 | cg23479922 | chr5 | 16179524 | 16179525 | MARCH11; | 4 |
| | | | | | RP11-1902.2 | |
| 5 | cg03738025 | chr6 | 104940819 | 104940820 | LINC00577 | 5 |
| 6 | cg26422458 | chr1 | 79006767 | 79006768 | ELTD1 | 6 |
| 7 | cg23746497 | chr6 | 104940793 | 104940794 | LINC00577 | 7 |
| 8 | cg03450948 | chr2 | 176188546 | 176188547 | HAGLR; | 8 |
| | | | | | HOXD1; | |
| | | | | | MIR7704 | |
| 9 | cg06782035 | chr5 | 16179026 | 16179027 | MARCH11; | 9 |
| | | | | | RP11-1902.2 | |
| 10 | cg10997627 | chr7 | 97733768 | 97733769 | TAC1 | 10 |
| 11 | cg18961681 | chr7 | 32428045 | 32428046 | · | 11 |
| 12 | cg15466862 | chr13 | 112068019 | 112068020 | SOX1 | 12 |
| 13 | cg03323696 | chr5 | 59893293 | 59893294 | PDE4D | 13 |
| 14 | cg25078444 | chr14 | 28765987 | 28765988 | FOXG1; | 14 |
| | | | | | FOXG1-AS1 | |
| 15 | cg13611347 | chr18 | 34159243 | 34159244 | NOL4 | 15 |
| 16 | cg12071888 | chr1 | 90729602 | 90729603 | · | 16 |
| 17 | cg15084543 | chr1 | 79006723 | 79006724 | ELTD1 | 17 |
| 18 | cg27252696 | chr6 | 100465064 | 100465065 | SIM1 | 18 |
| 19 | cg16065021 | chr12 | 127456109 | 127456110 | · | 19 |
| 20 | cg26890189 | chr19 | 47448045 | 47448046 | SLC8A2 | 20 |
| 21 | cg06335867 | chr7 | 8442695 | 8442696 | NXPH1 | 21 |
| 22 | cg04360793 | chr1 | 79006676 | 79006677 | ELTD1 | 22 |

In the following Table 2a the genomic sequence surrounding the CpG sites of the DMPs of Table 2 is provided in detail (with the CpG site being marked):

**Table 2a**

| **No.** | **CpG Site ID (DMP)** | **SEQ ID NO:** | **Sequence with marked CpG Site (underlined)** |
|---|---|---|---|
| 4 | cg23479922 | 4 | Gcggcggcgggggaggttgcgggggaggctcggcgtc cccgctctccgccccgcgacaccgactgccgccgtgg ccgccctc |
| 5 | cg03738025 | 5 | Cggtggccgcgcgccgcccggctgttccgcgtcccct tgccggctcacaatgtgctgaccctcccggccccgcc gcgcgcct |
| 6 | cg26422458 | 6 | Cgccgctgggcgggcgcggcagggtcccggatccgtc ctccgtgacgcgccggcttcctccggacactttccgg cctctttt |
| 7 | cg23746497 | 7 | Gtggtggcgacagcggattccgtgcccggtggccgcg cgccgcccggctgttccgcgtccccttgccggctcac aatgtgct |
| 8 | cg03450948 | 8 | Gctgggcggcggccctgggctgctttggctaacttgg ctgcggggcaggcgcgaggagcggcgcgccgaccggg ccgagcgg |
| 9 | cg06782035 | 9 | Ccggcgcgagctggagccgccaccggctgcctcgggg tctcgccgggccttacctgctccgcgccctggaagca gatcttgc |
| 10 | cg10997627 | 10 | Actgccggagccctttgagcatcttctgcagagaatc gcccggagacccaagcctcagcagttctttggattaa tgggcaaa |
| 11 | cg18961681 | 11 | Tagggagcgcgaatgggtctccgacagcaacgggagc agccggtggcgcctcaggctgcggtggcaacgagccc gactgcac |
| 12 | cg15466862 | 12 | Gcgcgcgctgcacatgaaggagcacccggattacaag taccggccgcgccgcaagaccaagacgctgctcaaga aggacaag |
| 13 | cg03323696 | 13 | Tgcggtccatggcgcgacagtacaggtagcgctcggt gtccgagtagccgcgatgccggacgcggccggtggcc ccgctcga |

| **No.** | **CpG Site ID (DMP)** | **SEQ ID NO:** | **Sequence with marked CpG Site (underlined)** |
|---|---|---|---|
| 14 | cg25078444 | 14 | Ccccagtcccggtttccccgcggtgccggagcccgga gctcgccgccgcccaggcctcaggaatcgagtaagta acccctgt |
| 15 | cg13611347 | 15 | Ccgacctgcaggcgcgctaagcgctccgggcgccgcg ctccgcgagggggagcagtgctcccaacccgcgaccc tccgcctc |
| 16 | cg12071888 | 16 | Aggagctgcaggctcgggtccccagccaggcggggga ctgcgggccgcccgcgctgcagggccgcgggtcaccg caggggc |
| 17 | cg15084543 | 17 | Ggagagcgcggcggagtgagtgcggctgtggacccgg gaccgggcgccgctgggcgggcgcggcagggtcccgg atccgtcc |
| 18 | cg27252696 | 18 | Gcgaggcgctcattggccaatagggctgagtgacacg agtcggccccgagccgccctccgcgcgcccaggctcc gggctctg |
| 19 | cg16065021 | 19 | Ggaccgcgggtgagcggtgggcaggtctcaggactct ctccggacgtgcggtccggggccgcgccgggtgctgg gcgcgcta |
| 20 | cg26890189 | 20 | Ctgcgtggtcgtcgtccaggatggtgacggtggccag cagcggcgccaccagccgccccttgggccgcccgccg ccgtccgg |
| 21 | cg06335867 | 21 | Cagtgttcaggccccggggtttcccagccgtagtggc cgccgccacagctgcgcgctttattgtctgctttcag tcgcaggt |
| 22 | cg04360793 | 22 | Ggagcgggaggcgtttcattggcggtggccgcagtgg tggcggtggcggagagcgcggcggagtgagtgcggct gtggaccc |

In preferred embodiments, the group of methylation sites additionally comprises 1, 2, 3, 4, 5, 6, 7, 8, 9, 10, 11, 12, 13, 14, 15, 16, 17, 18 or 19 DMPs of Table 2. For example, the group of methylation sites whose methylation level in genomic DNA derived from a sample of the subject is to be analyzed for determining the age group of the subject comprises the DMPs 1-3 (SEQ ID NO: 1-3) derived from Table 1/1a and any 1 DMP derived from Table 2/2a, e.g. any one of SEQ ID NO: 4, 5, 6, 7, 8, 9, 10, 11, 12, 13, 14, 15, 16, 17, 18, 19, 20, 21, or 22. In this embodiment, the group of methylation sites may accordingly comprise 4 methylation sites such as DMPs 1-4 (SEQ ID NO: 1-4), or DMPs 1-3 and 5 (SEQ ID NO: 1-3 and 5) etc.

In further embodiments, the group of methylation sites whose methylation level in genomic DNA derived from a sample of the subject is to be analyzed for determining the age group of the subject comprises the DMPs 1-3 (SEQ ID NO: 1-3) and any 2 DMPs derived from Table 2/2a, e.g. any 2 of SEQ ID NO: 4, 5, 6, 7, 8, 9, 10, 11, 12, 13, 14, 15, 16, 17, 18, 19, 20, 21, or 22. In this embodiment, the group may accordingly comprise 5 methylation sites such as DMPs 1-5 (SEQ ID NO: 1-5), or DMPs 1-3 and 5 and 6 (SEQ ID NO: 1-3 and 5 and 6) etc.

In particularly preferred embodiments the group of methylation sites whose methylation level in genomic DNA derived from a sample of the subject has to be analyzed for determining the age group of the subject comprises the DMPs 1-3 (SEQ ID NO: 1-3) derived from Table 1/1a and all DMPs derived from Table 2/2a, i.e. all of SEQ ID NO: 4, 5, 6, 7, 8, 9, 10, 11, 12, 13, 14, 15, 16, 17, 18, 19, 20, 21, and 22.

The DMPs mentioned herein, e.g. in Table 1, 1a, 2 or 2a are provided in the Illumina ID nomenclature. Information on the genomic location of the methylation sites, as well as data on associated experiments, studies and scientific references etc. can be derived, for example, from the website of Illumina (www.illumina.com). Accordingly, the identity of each DMP represented by each DMP loci identifier as depicted in the Tables is publicly available from the Illumina website, e.g., under reference to the DMP sites used in the Infinium HumanMethylation450 BeadChip kit.

Alternatively, a genome browser such as, for example, the genome browser of UCSC (http://genome-euro.ucsc.edu/), which provides a graphic scheme of the genomic location including additional information on the encoded sequences etc. can be used to derive molecular and positioning information on each DMP loci identifier as depicted in Tables 1, 1a, 2 or 2a. For instance, upon introducing the DMP's Illumina ID as shown in the Tables (cg...) into the genome browser, coordinates of the genomic location of the DMP can be retrieved. Coordinates of genomic location of the DMPs may accordingly be used for the design and synthesis of adjacent or binding polynucleotides, e.g., primer sequences, or for sequence analysis purposes etc. The Illumina ID nomenclature is constructed on CG loci designation which is based on actual or contextual sequences of each CpG locus and takes advantage of the sequences flanking the DMP. Thereby the ID is unaffected by reference genome changes or differences in genome versions. This allows for an unambiguous assignment of the ID to genomic coordinates.

Further, the Illumina Infinium HumanMethylation450 BeadChip array system (or any future and functionally equivalent variant thereof) may be used to further analyze or assess any of the DMPs shown in Tables 1, 1a, 2 or 2a.

According to specific embodiments, the methylation sites reflected by the panel of the invention or DMPs covered by the panel are located in more than one genomic location. For example, the methylation sites may be located on different chromosomes, different chromosomal arms, or in or close to different genes, as can be derived from the section "Gene" of Tables 1 and 2.

In preferred embodiments, the method according to the present invention comprises performing a statistical prediction of the affiliation of a subject to an age group as defined herein. The statistic prediction may comprise any suitable approach or algorithm known to the skilled person. In particularly preferred embodiments, the statistical prediction utilizes a machine learning approach trained on an appropriately representative training cohort. "Machine learning" as used herein typically relies on a two-step approach: first, a training phase; and second, a prediction phase. In the training phase, values of one or more parameters of the machine-learning model (MLM) are set using training techniques and training data. In the prediction phase, the trained MLM operates on measurement data. Building an MLM can include the training phase to determine the values of the parameters. Building an MLM can generally also include determining values of one or more hyperparameters. Typically, the values of one or more hyperparameters of the MLM are set and not altered during the training phase. Hence, the value of the hyperparameter can be altered in outer-loop iterations; while the value of the parameter of the MLM can be altered in inner-loop iterations. Sometimes, there can be multiple training phases, so that multiple values of the one or more hyperparameters can be tested or even optimized. The performance and accuracy of most MLMs are strongly dependent on the values of the hyperparameters. Example hyperparameters include: number of layers in a convolutional neural network; kernel size of a classifier kernel; input neurons of an ANN; output neurons of an ANN; number of neurons per layer; learning rate; etc. Various types and kinds of MLMs can be employed in the context of the present invention. For example, a novelty detector MLM / anomaly detector MLM, or a classifier MLM may be employed, e.g., a binary classifier. For example, a deep-learning (DL) MLM can be employed: here, features detected by the DL MLM may not be predefined, but rather may be set by the values of respective parameters of the model that can be learned during. As a general rule, various techniques can be employed for building the MLM. For example, typically, the type of the training can vary with the type of the MLM. Since the type of the MLMs can vary in different implementations, likewise, the type of the employed training can vary in different implementations. For example, an iterative optimization could be used that uses an optimization function that is defined with respect to one or more error signals. For example, a backpropagation algorithm can be employed.

The term "representative training cohort" as used in the context of the machine learning algorithms described above refers to a data set which has been successfully associated with age groups as defined herein. In particular, the term relates to a data set in which all parameters (including the target parameter to be predicted) are known and curated. Importantly, the ratio of cases per group is representative of the objective of the model to be trained.

A suitable example of a machine learning approach which may be used in the context of the statistical prediction according to the present invention is logistic regression. "Logistic regression" is a supervised machine learning algorithm which aims at a binary classification by predicting the probability of an event or observation. It typically analyzes the relationship between two or more independent variables and classifies the data subsequently into discrete classes. As such it may be used as part of predictive modeling for estimating the mathematical probability of an observation to belong to a specific category or not. Further information is known to the skilled person or can be derived from suitable literature references or textbooks such as Maalouf, 2011, International Journal of Data Analysis Techniques and Strategies, 3, 3, 281-299.

A further suitable example of a machine learning approach which may be used in the context of the statistical prediction according to the present invention is the Random Forest algorithm. "Random Forest" is a machine learning algorithm which combines the output of multiple decision trees to reach a single result. Decision trees typically start with a basic question which develop into more complex questions concerning a certain subject. These questions build decision nodes in a tree, acting as a means to split the data. Each question helps to arrive at a final decision. Decision trees typically seek to find the best split to subset the data, and are trained through, e.g. the Classification and Regression Tree (CART) algorithm. Metrics, such as Gini impurity, information gain, or mean square error (MSE), may be used to evaluate the quality of the split. Based on decision tree classifiers ensemble learning methods are used to aggregate the results of the classifiers. The Random Forest approach accordingly creates an uncorrelated forest of decision trees and selects a subset of available features in order to arrive at a certain prediction.

According to the present invention, the DNA molecule, i.e. genomic DNA molecule, for subsequent analysis may be derived from a subject's sample. The sample may be any suitable sample type or form. In preferred embodiments, the sample is a blood, e.g. plasma or serum, urine, feces, saliva, or liquid biopsy sample. In other embodiments, the sample may be a tissue, e.g., tissue biopsy, semen, liquor, or other sample. The present invention preferably envisages the use of liquid biopsy samples derived from blood/urine or other body fluids. Also envisaged is to make use of previously deposited samples. In certain embodiments the sample is a cell free DNA (cfDNA) sample. The term "cfDNA" as used herein refers to degraded DNA fragments released to body fluids such as blood plasma, urine, cerebrospinal fluid, etc., wherein the cfDNA typically has a short half-life and requires rapid processing and/or stabilization. The typical size of cfDNA fragments is about 165 bp. cfDNA comprises various forms of DNA freely circulating in body fluids such as circulating tumor DNA (ctDNA) or cell-free mitochondrial DNA (cf mtDNA). In further preferred embodiments the sample is a genomic DNA molecule sample, which can be derived, e.g., from a tissue obtained in a biopsy. It is particularly preferred that the sample is blood plasma sample.

The term "subject" as used herein refers to any human individual whose affiliation to an age group as defined herein shall be determined. The subject may, for example, be a suspect in a forensic case, a person of interest in a molecular analysis etc. The subject may be female or male (or diverse). The gender has no influence on the outcome of the age group prediction.

In a further preferred embodiment of the present invention, the method for determining the age group of a subject comprises the determination of the methylation level of the methylation sites by modifying the genomic DNA molecules to allow for determination of the methylation status of methylation sites, preferably by bisulfite-conversion or enzymatic conversion, and determining the sequence of molecules, thereby also determining the methylation status of the molecule.

The "modification of DNA molecules" may be based on any technology, which allows for the determination of the methylation status of methylation sites. It is preferred that the modification is a bisulfite-conversion or enzymatic conversion as described in detail herein. The method typically comprises a contacting of correspondingly modified DNA molecules with complementary polynucleotides resulting in a binding or hybridization reaction with complementary sequences, preferably probes as described in the following:
Generally, the methylation sites as defined herein, e.g. in Tables 1, 1a, 2 and 2a, are represented by nucleotide sequences which are capable of being bound by corresponding polynucleotide probes (e.g. probes complementary to the sequences of any one SEQ ID NO: 1 to 22, or complementary to reverse complements thereof). The term "represented by nucleotide sequences" as used herein means that the DMP itself is localized or embedded in a genomic context in the form of adjacent sequences at the 5' and 3' end. These contextual sequences (see e.g. the sequence of 40 nt 5' and 3' of the GC sites marked in any one of SEQ ID NO: 1 to 22), together with the genomic coordinate, represent the methylation site and allow for the provision of complementary or binding polynucleotides, e.g., in the form of probes. These complementary polynucleotides or probes may, for example, be capable of hybridizing to the nucleotide sequence representing the DMP. The term "complementary polynucleotide" as used herein includes both sense and antisense directions. Also, a mixture of complementary polynucleotides for both strands is envisaged.

The binding is a sequence-specific hybridization, which allows to specifically collect only DNA molecules which show a high degree of complementarity with the polynucleotide probes, e.g., 95%, 96%, 97%, 98%, 99%, 100%. The binding specificity may be adjusted by changes to the hybridization conditions, e.g., temperature, pH, ionic concentrations etc. The polynucleotide probes may have any suitable length, preferably a length of 50-150 nucleotides, more preferably of 80 or 120 nucleotides. The polynucleotide probes may be free floating or be tethered or linked to a support to allow for pulling down binding DNA molecules. Also, the alternative use of magnetic beads and a corresponding magnetic actuation approach is envisaged. Further contemplated are antibody-based techniques, as well as molecular approaches based on biotin/streptavidin interactions.

Subsequent to the contacting step, the bound DNA molecules may be enriched, i.e., only DNA molecules which are in fact specifically bound to the probes are kept or retained, whereas other, non-bound or not specifically bound DNA molecules or other components in a mixture are discarded. In principle, the enrichment is based on a hybridization of the complementary polynucleotides to the DNA molecule from a subject's sample and a subsequent pull-down of DNA molecules which are considered to be informative in the context of the present invention due to the differential methylation status of CpG sites in the DNA molecule. In principle, the DNA molecule to be enriched may be a non-modified DNA molecule or a modified DNA molecule, e.g., as described herein, preferably a DNA molecule which has been bisulfite-converted or enzymatically converted. The enriched DNA molecules may be used for further analysis steps or stored for future activities. Enriched DNA molecules may, in additional embodiments, further be amplified. Also contemplated is a spiking with specific indices that allow for a sequencing of the molecules.

As a subsequent step, which may in certain embodiments be optional, the method comprises a step of determining the sequence of the bound and/or enriched molecule. The sequence data of the bound and/or enriched and optionally previously modified DNA molecules may be obtained with any suitable technology, preferably in a high-throughput approach. This typically includes next-generation sequencing (NGS) or second-generation sequencing techniques. Such approaches comprise any sequencing method that determines the nucleotide sequence of either individual nucleic acid molecules or expanded clones for individual nucleic acid molecules in a highly parallel fashion. The sequencing may be performed according to any suitable massive parallel approach. Typical platforms include Illumina, Life Technologies Ion Proton, Oxford Nanopore Technologies, Pac Bio, Complete Genomics/BGI, Ultima, Element Biosciences, Singular Genomics, GeneReader, GenapSys, Solexa, Solid or Helicos Biosciences Heliscope systems. In certain embodiments, the sequencing may include a previous preparation of nucleic acids, the sequencing, as well as subsequent imaging and initial data analysis steps. Preparation steps may, for example, include amplification steps, e.g., PCR amplification, randomly breaking nucleic acids into smaller sizes and generating sequencing templates such as fragment templates. Spatially separated templates can, for example, be attached or immobilized at solid surfaces which allows for multiple sequencing reactions to be performed simultaneously. In typical examples, a library of nucleic acid fragments may be generated and adaptors containing universal priming sites may be ligated to the end of the fragments. Subsequently, the fragments can be denatured into single strands and captured by beads.

Suitable sequencing methods include, but are not limited to, cyclic reversible termination (CRT) or sequencing by synthesis (SBS) by Illumina, sequencing by ligation (SBL), single-molecule addition (pyrosequencing) or real-time sequencing. Exemplary platforms using CRT methods are Illumina/Solexa and HelicoScope. Exemplary SBL platforms include the Life/APG/SOLiD support oligonucleotide ligation detection. An exemplary pyrosequencing platform is Roche/454. Exemplary real-time sequencing platforms include the Pacific Biosciences platform and the Life/Visi-Gen platform. Other sequencing methods to obtain massively parallel nucleic acid sequence data include nanopore sequencing, sequencing by hybridization, nano-transistor array-based sequencing, scanning tunneling microscopy (STM) based sequencing, or nanowire-molecule sensor-based sequencing. Further details with respect to the sequencing approach would be known to the skilled person or can be derived from suitable literature sources such as Goodwin et al., Nature Reviews Genetics, 2016, 17, 333-351, or van Dijk et al., Trends in Genetics, 2014, 9, 418-426.

Correspondingly obtained data are provided in the form of sequencing reads which may be single-end or paired-end reads. Obtaining such sequencing data may further include the addition of assessment steps or data analysis steps.

Furthermore, the presently described methodology may be used with any suitable sequencing read length. It is preferred to make use of sequencing reads of a length of about 50 to about 170, e.g., 50, 60, 70, 80, 90, 100, 110, 120, 130, 140, 150, 160, 170 or more nucleotides or any value in between the mentioned values. Particularly preferred is paired end sequencing with 2 x 75 bp reads or with 2 x 150 bp reads.

The elucidation of the sequence of the DNA molecules allows to determine the methylation status of the bound and/or enriched molecule. For example, as described above, in the DNA sequencing, uracil, which is turned to thymine during amplification, is recognized as thymine during sequencing and on the opposite strand adenine is provided due to base pairing, while methylated cytidines are paired with guanines. C->U transitions at the unmethylated sites can accordingly be detected, e.g., by comparing the sequences with reference sequences or, for example, parallel sequencing results with non-bisulfite converted or non-enzymatic converted DNA molecules. Accordingly, in certain embodiments, the present invention envisages a double or parallel approach wherein DNA molecules from a sample are (i) modified by the bisulfite or enzymatic conversion; and (e.g., a portion of the sample) (ii) are not modified. Both groups of molecules are subsequently analyzed as described above, e.g., by determining the sequence. In further embodiments Methylation-Specific MLPA (multiplex ligation-dependent probe amplification) may be used. Finally, a comparison of both sequences may be performed to detect methylated positions. This may further be compared with sequences stored in databases comprising information on said DMPs or CpG sites. Examples of suitable databases include, for example, KEGG (Kanehisa and Goto, 2000, Nucleic Acids Research, 28, 1, 27-30), GO (Gene Ontology Consortium, 2004, Nucleic Acids Research, 32, Suppl 1, D258-D261).

In a further embodiment, the method comprises the reporting of the age group of the subject. A corresponding report may be provided in any suitable manner or form, e.g. as electronic file, as electronic file distributed or accessible over the internet, e.g. provided in cloud or deposited on a server, or web-based, e.g. provided on suitable web-site. Alternatively, the report may be provided in paper form. The report may be provided and thus drafted in a corresponding form, to a subject or another person associated with the subject, a scientist or group of scientists, a judicial authority, a hospital or clinic, a medical practitioner, a law enforcement authority, or a government office (including information relevant for these entities). The report may accordingly be redacted, modified, extended or adjusted to the above specified recipient.

In a further aspect, the present invention relates to a kit comprising, or alternatively consisting of, probes for detecting the DMPs corresponding to Illumina probe IDs cg05736768 (SEQ ID NO: 1), cg10741153 (SEQ ID NO: 2) and cg14002960 (SEQ ID NO: 3). In further embodiments, the kit may additionally comprise probes for detecting one or more DMPs of Table 2, preferably 1, 2, 3, 4, 5, 6, 7, 8, 9, 10, 11, 12, 13, 14, 15, 16, 17, 18 or 19 probes binding to cognate DMPs of Table 2. It is particularly preferred that the kit comprises one or more probes for detecting the DMPs corresponding to Illumina probe IDs cg23479922 (SEQ ID NO: 4), cg03738025 (SEQ ID NO: 5), cg26422458 (SEQ ID NO: 6), cg23746497 (SEQ ID NO: 7), cg03450948 (SEQ ID NO: 8), cg06782035 (SEQ ID NO: 9), cg10997627 (SEQ ID NO: 10), cg18961681 (SEQ ID NO: 11), cg15466862 (SEQ ID NO: 12), cg03323696 (SEQ ID NO: 13), cg25078444 (SEQ ID NO: 14), cg13611347 (SEQ ID NO: 15), cg12071888 (SEQ ID NO: 16), cg15084543 (SEQ ID NO: 17), cg27252696 (SEQ ID NO: 18), cg16065021 (SEQ ID NO: 19), cg26890189 (SEQ ID NO: 20), cg06335867 (SEQ ID NO: 21) or cg04360793 (SEQ ID NO: 22). The probes may have any suitable length. Preferably, they comprise 50 to 120 nucleotides, e.g., 50, 55, 60, 65, 70, 75, 80, 85, 90, 95, 100, 105, 110, 115, 120, 124, 130, 135, or 140 nucleotides, or any value in between the mentioned values. In particularly preferred embodiments, they have a length of about 80 or about 120 nucleotides. For example, the probe may be designed to hybridize to a DMP as shown in Table 1 or 2, e.g. according to conditions mentioned herein above. In a particularly preferred embodiment, the entire set of DMPs of Tables 1 and 2 may be represented by a number of 22 probes, e.g. probes having the sequences of SEQ ID NO: 1 to 22 or reverse complements thereof. The present invention also envisages a lower number of probes, e.g., if not all DMPs of Tables 1 and 2 are employed. Also envisaged is a higher number of probes, e.g., if a different target coverage is intended, or if additional DMPs not mentioned in Tables 1 or 2 are considered to be of interest, the kit according to the present invention may comprise one or more additional probes which may be further included. Such additional probes may, for example, be identified in further future analyses or be derived from known age methylation groups.

The probes of the kit according to the present invention may, in certain embodiments, be linked or tethered to a solid support such as an array structure or the like. Also, a link, e.g., via biotin-streptavidin interaction, with magnetic beads and a corresponding actuation and fixation by magnetic forces is envisaged. Alternatively, the probes may be free floating.

In a further aspect, the present invention also envisages the use of DMPs defined herein, preferably of DMPs corresponding to Illumina probe IDs cg05736768 (SEQ ID NO: 1), cg10741153 (SEQ ID NO: 2) and cg14002960 (SEQ ID NO: 3) for the determining the age group of a subject. In preferred embodiments, the group further comprises one or more of the DMPs corresponding to Illumina probe IDs cg23479922 (SEQ ID NO: 4), cg03738025 (SEQ ID NO: 5), cg26422458 (SEQ ID NO: 6), cg23746497 (SEQ ID NO: 7), cg03450948 (SEQ ID NO: 8), cg06782035 (SEQ ID NO: 9), cg10997627 (SEQ ID NO: 10), cg18961681 (SEQ ID NO: 11), cg15466862 (SEQ ID NO: 12), cg03323696 (SEQ ID NO: 13), cg25078444 (SEQ ID NO: 14), cg13611347 (SEQ ID NO: 15), cg12071888 (SEQ ID NO: 16), cg15084543 (SEQ ID NO: 17), cg27252696 (SEQ ID NO: 18), cg16065021 (SEQ ID NO: 19), cg26890189 (SEQ ID NO: 20), cg06335867 (SEQ ID NO: 21), or cg04360793 (SEQ ID NO: 22), for the determining the age group of a subject. In additional embodiments, the present invention also envisages the use of a kit as defined herein above, for the binding and/or analysis of the DMPs as defined in Table 1, optionally as defined in Tables 1 and 2. The kit is preferably used for determining the age group of a subject as described herein.

In yet another aspect, the present invention relates to a method of forensic DNA analysis of samples derived from male subjects. This method may, for example, be used for the determination of male subjects to age groups as defined herein and advantageously allows to include or exclude subjects from a list of suspects due to association to a certain age group. Further the method may allow for a narrowing of a suspect group to a younger (e.g., sons) or older (e.g., father, grandfather) cohort of a family, which advantageously facilitates law enforcement. In preferred embodiments, the method comprises the performance of Y-chromosome short tandem repeat polymorphism (Y-STR) analysis of a subject's sample and determining the methylation level of a group of methylation sites in a genomic DNA derived from the subject's sample. The "Y-chromosome short tandem repeat polymorphism (Y-STR) analysis" is an approach which is directed to the analysis of short tandem repeat (STR) markers located on the Y chromosome. The Y-STR analysis is typically capable of detecting the presence of very small amounts of male DNA of one or multiple donors and resulting genetic profiles can be compared to known reference samples. The Y-STR analysis may, for example, be applied in crime scene investigations and can exclude male suspects from involvement in crime, identify the paternal lineage of male perpetrators, highlight multiple male contributors to a trace, and provide investigative leads for finding unknown male perpetrators. It may also be used in paternity disputes or paternal kinship testings. The approach typically comprises the analysis of one or more of the markers DYS19, DYS385, DYS389I, DYS389II, DYS390, DYS391, DYS392, DYS393, DYS437, DYS438, DYS439, DYS448, DYS449, DYS456, DYS458, DYS460, DYS481, DYS505, DYS518, DYS522, DYS526, DYS533, DYS547, DYS549, DYS570, DYS576, DYS612, DYS626, DYS627 DYS635, DYS643, GATA H4, DYF387S1, DYF399S1, DYF403S1, and DYF404S1. In preferred embodiments, the analysis of these markers (or other suitable makers known to the skilled person or to be developed in the future) may be combined with the analysis of the group of methylation sites as defined herein, in particular with the group of DMPs corresponding to Illumina probe IDs cg05736768 (SEQ ID NO: 1), cg10741153 (SEQ ID NO: 2) and cg14002960 (SEQ ID NO: 3). In further embodiments, the analysis may additionally comprise the analysis of one or more of the DMPs corresponding to Illumina probe IDs cg23479922 (SEQ ID NO: 4), cg03738025 (SEQ ID NO: 5), cg26422458 (SEQ ID NO: 6), cg23746497 (SEQ ID NO: 7), cg03450948 (SEQ ID NO: 8), cg06782035 (SEQ ID NO: 9), cg10997627 (SEQ ID NO: 10), cg18961681 (SEQ ID NO: 11), cg15466862 (SEQ ID NO: 12), cg03323696 (SEQ ID NO: 13), cg25078444 (SEQ ID NO: 14), cg13611347 (SEQ ID NO: 15), cg12071888 (SEQ ID NO: 16), cg15084543 (SEQ ID NO: 17), cg27252696 (SEQ ID NO: 18), cg16065021 (SEQ ID NO: 19), cg26890189 (SEQ ID NO: 20), cg06335867 (SEQ ID NO: 21), or cg04360793 (SEQ ID NO: 22), for the determining the age group of a subject.

The following figures and examples are provided for illustrative purposes. It is thus understood that the figures and examples are not to be construed as limiting. The skilled person in the art will clearly be able to envisage further modifications of the principles laid out herein.

### EXAMPLES

### 1. PANEL ANALYSIS

32 healthy normals (23 males and 9 females) were evaluated using a methylation panel covering >4000 CpG sites (see FIG. 1) revealing a distinct set of CpG markers to discriminate a young from an old cohort. Methylation assessment at those sites have been performed by targeted hybridization assays using specific primer sets detecting both, the methylated and the unmethylated CpG site.

The healthy normals were categorized in a young (<40 years old, 12 males & 5 females) and an old cohort (> 63 years old, 11 males & 4 females). Differential methylation profiles of each CpG site, using m-values (Du et al., 2010, BMC Bioinformatics, 11, 587) as a measure, were bioinformatically assessed using two-sided Kolmogorov-Smirnov (KS)-testing revealing three CpG sites (see Table 1, 1a and FIG. 3) which significantly (significance level: p < 0.00001) differed between the young and the old cohort.

Randomized repetitions of the two-sided KS-testing was performed to correct for multiple testing and confirming the statistical significance of the three CpG sites identified.

### 2. HIERARCHICAL WARD CLUSTERING

Hierarchical ward clustering on CpG sites with significant KS-test between the young and old cohort was performed and showed positive correlation of the three CpG sites with age (see also FIG. 3), therefore indicating a potential to improve the age determinations of the prior art based on other DNA methylation sites.

## Claims

1. A method for determining the age group of a subject, comprising determining the methylation level of a group of methylation sites in a genomic DNA derived from a sample of the subject, wherein said group of methylation sites comprises differentially methylated positions (DMPs) corresponding to Illumina probe IDs cg05736768 (SEQ ID NO: 1), cg10741153 (SEQ ID NO: 2) and cg14002960 (SEQ ID NO: 3).

2. The method of claim 1, wherein the determination of the age group of the subject comprises performing a statistical prediction.

3. The method of claim 2, wherein said statistical prediction utilizes a machine learning approach such as logistic regression or random forest classification trained on an appropriately representative training cohort.

4. The method of any one of claims 1 to 3, wherein the determination of the age group of the subject comprises a distinction between a younger person, preferably of < 40 years and an older person, preferably of > 60 years, more preferably of > 63 years.

5. The method of any one of claims 1 to 4, wherein said sample is a blood, urine, feces, saliva, or liquid biopsy sample, preferably a blood plasma sample.

6. The method of any one of claims 1 to 5, wherein the group of methylation sites additionally comprises one or more, namely 1, 2, 3, 4, 5, 6, 7, 8, 9, 10, 11, 12, 13, 14, 15, 16, 17, 18 or 19 DMPs of Table 2.

7. The method of any one of claims 1 to 6, wherein the determination of the methylation level of the methylation sites comprises modifying the genomic DNA molecules to allow for determination of the methylation status of methylation sites, preferably by bisulfite-conversion or enzymatic conversion, and determining the sequence of the molecules, thereby also determining the methylation status of the molecules.

8. The method of any one of claims 1 to 7, comprising the step of reporting the age group of the subject, preferably in an electronic form.

9. A kit comprising probes for detecting the differentially methylated positions (DMPs) corresponding to Illumina probe IDs cg05736768 (SEQ ID NO: 1), cg10741153 (SEQ ID NO: 2) and cg14002960 (SEQ ID NO: 3).

10. The kit of claim 9, additionally comprising one or more probes for detecting one or more DMPs of Table 2, preferably 1, 2, 3, 4, 5, 6, 7, 8, 9, 10, 11, 12, 13, 14, 15, 16, 17, 18 or 19 probes binding to DMPs of Table 2, more preferably one or more probes for detecting the DMPs corresponding to Illumina probe IDs cg23479922 (SEQ ID NO: 4), cg03738025 (SEQ ID NO: 5), cg26422458 (SEQ ID NO: 6), cg23746497 (SEQ ID NO: 7), cg03450948 (SEQ ID NO: 8), cg06782035 (SEQ ID NO: 9), cg10997627 (SEQ ID NO: 10), cg18961681 (SEQ ID NO: 11), cg15466862 (SEQ ID NO: 12), cg03323696 (SEQ ID NO: 13), cg25078444 (SEQ ID NO: 14), cg13611347 (SEQ ID NO: 15), cg12071888 (SEQ ID NO: 16), cg15084543 (SEQ ID NO: 17), cg27252696 (SEQ ID NO: 18), cg16065021 (SEQ ID NO: 19), cg26890189 (SEQ ID NO: 20), cg06335867 (SEQ ID NO: 21), cg04360793 (SEQ ID NO: 22).

11. A method of forensic DNA analysis of samples derived from male subjects, comprising the performance of Y-chromosome short tandem repeat polymorphism (Y-STR) analysis of a subject's sample and determining the methylation level of a group of methylation sites in a genomic DNA derived from a sample of the subject, wherein said group of methylation sites comprises differentially methylated positions (DMPs) corresponding to Illumina probe IDs cg05736768 (SEQ ID NO: 1), cg10741153 (SEQ ID NO: 2) and cg14002960 (SEQ ID NO: 3), wherein the determination of the methylation level allows for the determination of the age group of said subject.

12. The method of claim 11, wherein the group of methylation sites additionally comprises one or more, namely 1, 2, 3, 4, 5, 6, 7, 8, 9, 10, 11, 12, 13, 14, 15, 16, 17, 18 or 19 DMPs of Table 2.

13. Use of differentially methylated positions (DMPs) corresponding to Illumina probe IDs cg05736768 (SEQ ID NO: 1), cg10741153 (SEQ ID NO: 2) and cg14002960 (SEQ ID NO: 3), and optionally additionally of one or more of the DMPs corresponding to Illumina probe IDs cg23479922 (SEQ ID NO: 4), cg03738025 (SEQ ID NO: 5), cg26422458 (SEQ ID NO: 6), cg23746497 (SEQ ID NO: 7), cg03450948 (SEQ ID NO: 8), cg06782035 (SEQ ID NO: 9), cg10997627 (SEQ ID NO: 10), cg18961681 (SEQ ID NO: 11), cg15466862 (SEQ ID NO: 12), cg03323696 (SEQ ID NO: 13), cg25078444 (SEQ ID NO: 14), cg13611347 (SEQ ID NO: 15), cg12071888 (SEQ ID NO: 16), cg15084543 (SEQ ID NO: 17), cg27252696 (SEQ ID NO: 18), cg16065021 (SEQ ID NO: 19), cg26890189 (SEQ ID NO: 20), cg06335867 (SEQ ID NO: 21), or cg04360793 (SEQ ID NO: 22), for the determining the age group of a subject.

14. The use of claim 13, wherein the determination of the age group of a subject is performed within a process of forensic DNA analysis comprising the performance of Y-chromosome short tandem repeat polymorphism (Y-STR) analysis.
